# EUROPEAN PATENT APPLICATION

(11) **EP 4 799 588 A1**
(43) Date of publication of application: **02.09.2026**
(21) Application number: 24910001.7
(22) Date of filing: 10.09.2024
(51) Int. Cl.: A61F 2/01, A61B 17/22

(54) **THROMBUS FILTERING DEVICE**

(30) Priority: 25.12.2023 CN 202311796401
(71) Applicant: Shanghai Shenqi Medical Technology Co., Ltd., Shanghai 201201 (CN)
(72) Inventor: GU, Yingying, Shanghai 201201 (CN); DENG, Hongxing, Shanghai 201201 (CN); ZHOU, Guolei, Shanghai 201201 (CN); WANG, Sen, Shanghai 201201 (CN)
(74) Representative: Zacco Sweden AB
(86) International application number: PCT/CN2024/118045
(87) International publication number: WO 2025/139055

(57) **Abstract**

A thrombus filtering device, comprising: an outer sheath tube (201), a middle sheath tube (202), a proximal filter (204), an inner sheath tube (203) and a distal filter (205). The middle sheath tube (202) comprises a pushing tube (2021) and a steerable tube, the distal end of the pushing tube (2021) being connected to the proximal end of the steerable tube, and the proximal filter (204) being connected to the pushing tube (2021). The outer sheath tube (201) is movably sleeved outside the middle sheath tube (202). The distal filter (205) is connected to the distal end of the inner sheath tube (203); and the inner sheath tube (203) movably penetrates through the middle sheath tube (202), and comprises a proximal delivery tube (2033) and a distal fixing tube (2032) which are connected to each other, the distal fixing tube (2032) being a metal tube and comprising a spring tube section (20321).

## Description

### RELATED APPLICATIONS

This application claims priority to Chinese Patent Application No. 202311796401.8, filed on December 25, 2023, and entitled "Thrombus Filtering Device", the relevant content of which is incorporated herein by reference.

### TECHNICAL FIELD

The present application relates to the technical field of medical devices, and in particular to a thrombus filtering device.

### BACKGROUND

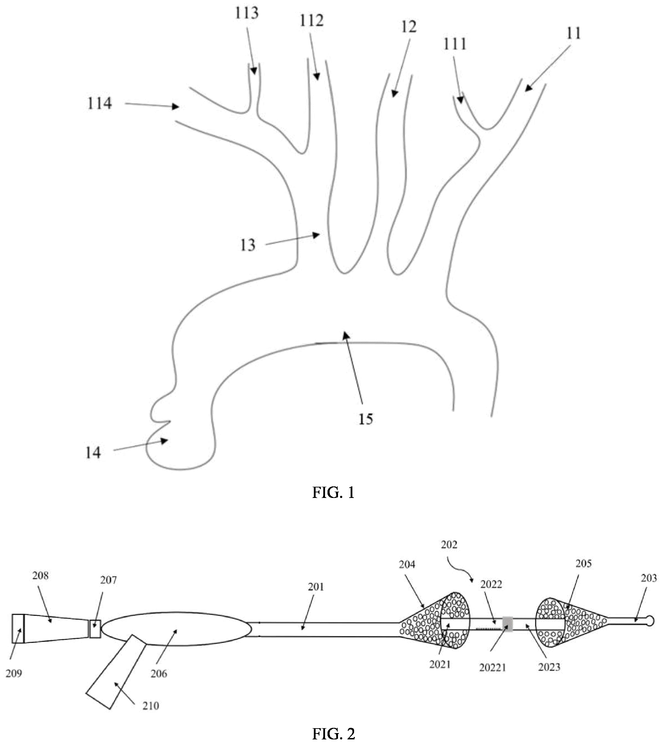

In the human body, brain cells have a very high demand for oxygen in blood. A total of four arteries deliver oxygen-containing blood to the brain to supply blood thereto. Referring to FIG. 1, they are respectively the left vertebral artery 111, the left common carotid artery 12, the right vertebral artery 113, and the right common carotid artery 112.

With the increasing prevalence of surgeries involving the heart and aorta, e.g., transcatheter aortic valve replacement, mitral annuloplasty, and other procedures, some materials such as platelets, fibrinogen, fibrocartilage, bacterial clots, and other small tissue fragments may be directly or indirectly detached during the course of these procedures.

These detached materials may flow with blood along the arteries toward the brain, and when an artery is blocked by these materials, tissue ischemia may be caused, further triggering cerebral vascular thrombosis, leading to myocardial infarction, stroke, or even death. Currently, the diseases related to cerebral vascular thrombosis have become very common complications in the aortic valve 14 (as shown in FIG. 1) procedures and other cardiac procedures.

Therefore, in some related technical solutions, in order to reduce these complications of cerebral vascular thrombosis caused by cardiac procedures, a thrombus filtering device with filters is implanted into blood vessels supplying blood to the brain to prevent cerebral thrombosis. A typical thrombus filtering device includes a proximal filter and a distal filter, with the proximal filter positioned in the brachiocephalic artery 13 and the distal filter positioned in the left common carotid artery 12.

However, the length, angle, and other conditions of arterial vessels vary among different patients. In a thrombus filter device in the prior art, the relative distance between the proximal filter and the distal filter is fixed, thereby making it difficult to positon both of them at suitable vascular sites in the arterial vessels for different patients.

### SUMMARY

In view of this, there is a need to provide a thrombus filtering device to address the issue in the prior art that the relative distance between a proximal filter and a distal filter of the thrombus filtering device is fixed, thereby making it difficult to place both of them at suitable vascular positions in arterial vessels for different patients.

The present application provides a thrombus filtering device including: an outer sheath tube, an intermediate sheath tube, a proximal filter, an inner sheath tube, and a distal filter;
wherein the intermediate sheath tube includes a push tube and an articulating sheath, a distal end of the push tube is connected to a proximal end of the articulating sheath, the proximal filter is connected to the push tube, and the outer sheath tube is movably sleeved outside the intermediate sheath tube;
the distal filter is connected to a distal end of the inner sheath tube, and the inner sheath tube is movably inserted in the intermediate sheath tube;
the inner sheath tube includes a proximal delivery tube and a distal fixing tube which are connected to each other, and the distal fixing tube is a metal tube; and the distal fixing tube includes a spring tube section.

In an embodiment, the distal fixing tube includes a cutting tube section. The cutting tube section is provided with a plurality of cutting slots sequentially arranged along an axial direction.

In an embodiment, the proximal filter includes a first elastic metal framework and a first polymer film. The first polymer film includes a first porous filter membrane that covers the first elastic metal framework. The first porous filter membrane has a collapsed state and an expanded state. In the expanded state, the first porous filter membrane tapers along a direction from a distal end to a proximal end.

In an embodiment, the distal filter includes a second elastic metal framework and a second polymer film. The second polymer film includes a second porous filter membrane that is connected to the second elastic metal framework, the second porous filter membrane has a collapsed state and an expanded state. In the expanded state, the second porous filter membrane tapers along a direction from a proximal end to a distal end.

In an embodiment, the first elastic metal framework includes a first support ring, a first support wire, and a first fixing wire. A distal end of the first porous filter membrane is connected to and fitted in shape with the first support ring; the first fixing wire is attached and fixed to the push tube, a proximal end of the first support wire is connected to the first fixing wire, and a distal end of the first support wire is connected to the first support ring.

In an embodiment, the first polymer film further includes a support membrane. The support membrane is disposed around and attached to a proximal end of the first porous filter membrane.

In an embodiment, the first polymer film further includes an inner sleeve section. The inner sleeve section is sleeved on and attached to the push tube, and the proximal end of the first porous filter membrane is sleeved on and attached to the inner sleeve section.

In an embodiment, the first polymer film further includes an outer sleeve section. The outer sleeve section is sleeved on and attached to the proximal end of the first porous filter membrane.

In an embodiment, the inner sheath tube includes a proximal delivery tube and a distal fixing tube which are connected, and the distal fixing tube is a metal tube and is configured to fix the distal filter.

In an embodiment, the inner sheath tube includes a tip tube, the tip tube is a polymer tube, and a proximal end of the tip tube is connected to a distal end of the distal fixing tube;
a distal end of the second elastic metal framework is connected to a proximal end of the second porous filter membrane and supports a shape of the proximal end of the second porous filter membrane, a distal end of the second porous filter membrane is connected to the tip tube, and the second elastic metal framework is connected to the distal fixing tube.

In an embodiment, the second polymer film includes a tip cap. The tip cap is connected to the distal end of the second porous filter membrane, and the tip cap wraps a distal end of the tip tube.

In an embodiment, the second elastic metal framework includes a second support ring, a second support wire, and a second fixing wire. The proximal end of the second porous filter membrane is connected to and fitted in shape with the second support ring; the second fixing wire is attached and fixed to the distal fixing tube, a proximal end of the second support wire is connected to the second fixing wire, and a distal end of the second support wire is connected to the second support ring.

During use of the thrombus filtering device described above, first the thrombus filtering device is adjusted to a retrieval state, and then it is inserted through a radial artery puncture to extend into a right subclavian artery and a brachiocephalic artery. Then, by withdrawing the outer sheath tube, the proximal filter is exposed from a distal end of the outer sheath tube so as to be positioned in the brachiocephalic artery. Further, by moving the inner sheath tube distally relative to the intermediate sheath tube and extending the same into an aortic arch, the proximal filter is exposed from a distal end of the articulating sheath. Then, by bending the articulating sheath, the distal filter extending out of the articulating sheath enters from the aortic arch into a left common carotid artery as the articulating sheath bends, while the proximal filter can remain in the brachiocephalic artery. Since the inner sheath tube can move relative to the intermediate sheath tube, the relative distance between the distal filter arranged on the inner sheath tube and the proximal filter arranged on the intermediate sheath tube can be adjusted, and thereby the relative distance between the proximal filter and the distal filter can be adjusted. In this way, the thrombus filtering device described above enables the adjustment of the relative distance between the proximal filter and the distal filter according to actual conditions of blood vessels of different patients, so that both the proximal filter and the distal filter can be quickly and accurately positioned at suitable vascular sites, thereby allowing the thrombus filtering device described above to flexibly adapt to arterial vessels of different patients. Moreover, since the inner sheath tube can move relative to the intermediate sheath tube, the proximal filter and the distal filter can move relatively independently, thereby facilitating independently adjusting the positions of the proximal filter and the distal filter, respectively. During a process in which the spring tube section is retrieved into the articulating sheath, it can adapt to a bent portion of the articulating sheath through its elastic bending deformation, thereby facilitating retrieval of the inner sheath tube carrying the distal filter into the articulating sheath.

The above description is merely a summary of the technical solutions of the present application. In order to clearly illustrate the technical solutions of the present application for implementation according to the contents of the specification, as well as to make the above and other objectives, features, and advantages of the present application more apparent and comprehensible, specific embodiments of the present application are described below.

### BRIEF DESCRIPTION OF THE DRAWINGS

To illustrate the technical solutions in the embodiments of the present application or in the conventional technology more clearly, the drawings required for describing the embodiments or the conventional technology will be briefly introduced below. Apparently, the drawings in the following description are merely some embodiments of the present application, and for persons of ordinary skill in the art, other drawings can also be obtained according to the disclosed drawings without any creative effort.
FIG. 1 is a schematic structural view of the aortic arch of a human body.
FIG. 2 is a schematic structural view of a thrombus filtering device according to an embodiment.
FIG. 3 is a schematic view of inner and outer layer relationships among various tubes and filters of the thrombus filtering device of FIG. 2.
FIG. 4 is a schematic view of the thrombus filtering device of FIG. 2 when an articulating sheath is bent once.
FIG. 5 is a schematic view of the thrombus filtering device of FIG. 2 when the articulating sheath is bent twice.
FIG. 6 is a schematic view of the thrombus filtering device of FIG. 2 in a use state when implanted in a human body.
FIG. 7 is a schematic view of the thrombus filtering device of FIG. 2 in a retrieval state.
FIG. 8 is a schematic structural view of a first elastic metal framework according to an embodiment.
FIG. 9 is a partial enlarged view of area A in FIG. 8.
FIG. 10 is a schematic structural view of a first porous filter membrane according to an embodiment.
FIG. 11 is a schematic view of a connection relationship between the first porous filter membrane of FIG. 10 in an expanded state and a support membrane.
FIG. 12 is a schematic structural view of a first porous filter membrane, an inner sleeve section, an outer sleeve section, and a fixing wire according to an embodiment.
FIG. 13 is a schematic view of inner and outer layer relationships among various components of a proximal filter according to an embodiment.
FIG. 14 is a schematic structural view of a distal fixing section of an inner sheath tube according to an embodiment.
FIG. 15 is a schematic structural view of an inner sheath tube according to an embodiment.
FIG. 16 is a schematic view of a connection relationship between an inner sheath tube and a distal filter according to an embodiment.
FIG. 17 is a schematic view of inner and outer layer relationships among various components of the inner sheath tube of FIG. 16.
FIG. 18 is a schematic structural view of a second elastic metal framework according to an embodiment.
FIG. 19 is a partial enlarged view of area B in FIG. 18.
FIG. 20 is a schematic structural view of a second porous filter membrane according to an embodiment.
FIG. 21 is a schematic view of the second porous filter membrane of FIG. 20 in an expanded state.

### DESCRIPTION OF REFERENCE NUMERALS

11: left subclavian artery; 12: left common carotid artery; 13: brachiocephalic artery; 14: aortic valve; 111: left vertebral artery; 112: right common carotid artery; 113: right vertebral artery; 114: right subclavian artery;
201: outer sheath tube;
202: intermediate sheath tube; 2021: push tube; 2022: first tube; 2023: second tube; 20221: connecting piece; 20231: bend-adjusting pull wire;
203: inner sheath tube:
2031: tip tube;
2032: distal fixing tube; 20321: spring tube section; 20322: connecting portion; 20323: cutting tube section; 20324: cutting slot;
2033: proximal delivery tube;
204: proximal filter;
2041: first elastic metal framework; 20411: first support ring; 20411A: connection point; 20411B: connection point; 20412: first support wire; 20413: first fixing wire;
2042: first polymer film; 20421: first porous filter membrane; 20422: support membrane; 20423: inner sleeve section; 20424: outer sleeve section;
205: distal filter;
2051: second elastic metal framework; 20511: second support ring; 20511A: connection point; 20511B: connection point; 20512: second support wire; 20513: second fixing wire;
2052: first polymer film; 20521: second porous filter membrane; 2053: tip cap;
206: first handle; 207: sheath pushing knob; 208: second handle; 209: filter pushing knob; 210: bend-adjusting control knob.

### DETAILED DESCRIPTION

The technical solutions of the embodiments of the present application will be clearly and completely described below with reference to the accompanying drawings in the embodiments of the present application. Apparently, only a part of the embodiments, not all the embodiments of the present application, are described. All other embodiments obtained, based on the embodiments described in the present application, by those skilled in the art without paying creative efforts shall fall within the protection scope of the present application.

The following describes in detail the embodiments of technical solutions in this application with reference to the accompanying drawings. The following embodiments are merely intended for a clearer description of the technical solutions of this application and therefore are used as just examples which do not constitute any limitations on the protection scope of this application.

Unless otherwise defined, all technical and scientific terms used herein shall have the same meanings as commonly understood by those skilled in the art to which this application belongs. The terms used herein are intended to merely describe the specific embodiments rather than to limit this application. The terms "include(comprise)" and "have" and any other variations thereof in the specification, claims and brief description of drawings of this application are intended to cover non-exclusive inclusions.

In the description of the embodiments of this application, the terms "first", "second" and the like are merely intended to distinguish between different objects, and shall not be understood as an indication or implication of relative importance or implicit indication of the number, specific sequence or dominant-subordinate relationship of the technical features indicated. In the description of this application, "a plurality of" means at least two unless otherwise specifically stated.

The term "embodiment" described herein means that specific features, structures, or characteristics in combination with descriptions of the embodiments may be incorporated in at least an embodiment of this application. The word "embodiment" appearing in various places in the specification does not necessarily refer to the same embodiment or an independent or alternative embodiment that is exclusive of other embodiments. It is explicitly or implicitly understood by persons skilled in the art that the embodiments described herein may be combined with other embodiments.

In the description of the embodiments of this application, the term "and/or" is only an associative relationship for describing associated objects, indicating that three relationships may be present. For example, A and/or B may indicate the following three cases: presence of only A, presence of both A and B, and presence of only B. In addition, the character "/" in this specification generally indicates an "or" relationship between contextually associated objects.

In the description of the embodiments of this application, the term "a plurality of" means more than two (including two). Similarly, "multiple groups" means more than two groups (including two groups), and "multiple pieces" means more than two pieces (including two pieces).

In the description of the embodiments of this application, the orientations or positional relationships indicated by the technical terms "center", "longitudinal", "transverse", "length", "width", "thickness", "upper", "lower", "front", "rear", "left", "right", "vertical", "horizontal", "top", "bottom", "inside", "outside", "clockwise", "counterclockwise", "axial", "radial", "circumferential", and the like are based on the orientations or positional relationships as shown in the accompanying drawings. These terms are merely for ease and brevity of description of the embodiments of this application rather than indicating or implying that the means or components mentioned must have specific orientations or must be constructed or manipulated according to specific orientations, and therefore shall not be construed as any limitations on the embodiments of this application.

In the description of the embodiments of this application, unless otherwise specified and defined explicitly, the technical terms "mount", "connect", "join", and "fasten" should be understood in a broad sense. For example, they may refer to a fixed connection, a detachable connection, or an integrated connection, may refer to a mechanical connection or an electrical connection, and may refer to a direct connection, an indirect connection through an intermediate medium, an internal connection between two elements, or an interaction between two elements. The specific meanings of these terms in the present application can be understood based on specific circumstances by those of ordinary skills in the art.

In the description of the embodiments of the present application, the terms "proximal" and "distal" are intended to refer to relative orientations, relative positions and directions of components or actions with respect to each other, as viewed by a physician operating the medical device. Without wishing to be limiting, a "proximal end" usually refers to an end closer to the operator, and a "distal end" to an end first enters the body of a patient, during normal operation of the medical device.

Referring to FIG. 1 , an aortic arch 15 has three blood vessels thereon, which are the left subclavian artery 11, the left common carotid artery 12, and the brachiocephalic artery 13 in sequence. The left subclavian artery 11 has a branch of the left vertebral artery 111, the brachiocephalic artery 13 has a bifurcation communicating with the right common carotid artery 112 and the right vertebral artery 113, and the brachiocephalic artery 13 further communicates with the right subclavian artery 114. Since some substances such as platelets, fibrinogen, fibrocartilage, bacterial clots, and other small tissue fragments, etc. will be detached during the course of aortic valve 14 replacement or other types of cardiac procedures, these substances will flow along the blood vessels above the aortic arch 15. The left vertebral artery 111, the right vertebral artery 113, the left common carotid artery 12, and the right common carotid artery 112, which are the blood vessels located above the aortic arch 15, supply blood to the brain. If these substances flow along these four blood vessels toward cerebral blood vessels, various complications will be caused.

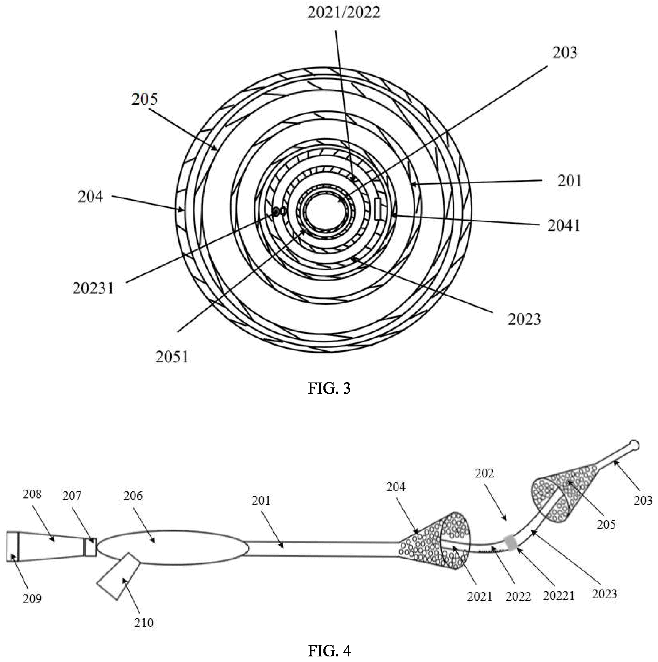

Referring to FIG. 2 to FIG. 7 in combination, an embodiment of the present application provides a thrombus filtering device. The thrombus filtering device includes: an outer sheath tube 201, an intermediate sheath tube 202, a proximal filter 204, an inner sheath tube 203, and a distal filter 205.

The intermediate sheath tube 202 includes a push tube 2021 and an articulating sheath, and a distal end of the push tube 2021 is connected to a proximal end of the articulating sheath. The proximal filter 204 is connected to the push tube 2021. The outer sheath tube 201 is movably sleeved outside the intermediate sheath tube 202 so as to sleeve or to expose the proximal filter 204. Specifically, withdrawing the outer sheath tube 201 proximally relative to the intermediate sheath tube 202 can expose the proximal filter 204 from a distal end of the outer sheath tube 201; conversely, moving the outer sheath tube 201 distally can make the the outer sheath tube 201 sleeved outside the proximal filter 204. The push tube 2021 is configured to push the articulating sheath, so as to realize the overall movement of the intermediate sheath tube 202.

The distal filter 205 is connected to a distal end of the inner sheath tube 203. The inner sheath tube 203 is movably inserted in the intermediate sheath tube 202, so as to drive the distal filter 205 to extend out of or retract into the articulating sheath. Specifically, moving the inner sheath tube 203 distally relative to the intermediate sheath tube 202 can expose the proximal filter 204 from a distal end of the articulating sheath; conversely, moving the inner sheath tube 203 proximally can retract the proximal filter 204 into the articulating sheath.

The articulating sheath can be bent, so that the distal filter 205 extending out of the articulating sheath can be located in the left common carotid artery 12, and the proximal filter 204 can be located in the brachiocephalic artery 13. Specifically, by providing a bend-adjusting pull wire 20231, a distal end of the bend-adjusting pull wire 20231 is connected to the articulating sheath, and the bend-adjusting pull wire 20231 passes through the articulating sheath and the push tube 2021. Pulling the bend-adjusting pull wire 20231 from a proximal end of the bend-adjusting pull wire 20231 can drive the articulating sheath to bend.

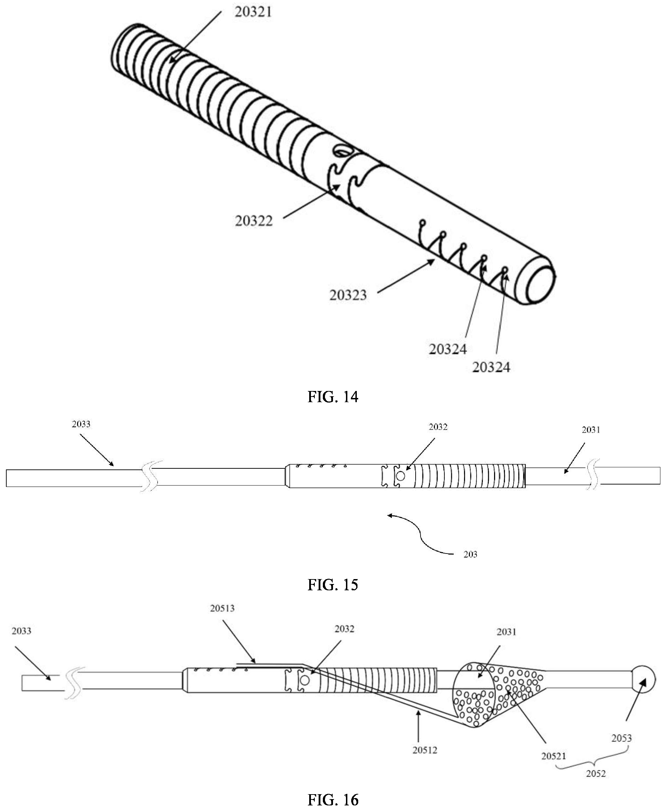

Referring to FIG. 14 to FIG. 16 in combination, the inner sheath tube 203 includes a proximal delivery tube 2033 and a distal fixing tube 2032 which are connected to each other, and a distal end of the proximal delivery tube 2033 is connected to a proximal end of the distal fixing tube 2032. The distal fixing tube 2032 is a metal tube and is configured to fix the distal filter 205, and specifically, can be connected to the distal filter 205 by means of welding, bonding, etc. The distal fixing tube 2032 includes a spring tube section 20321, and the spring tube section 20321 can be a helical spring.

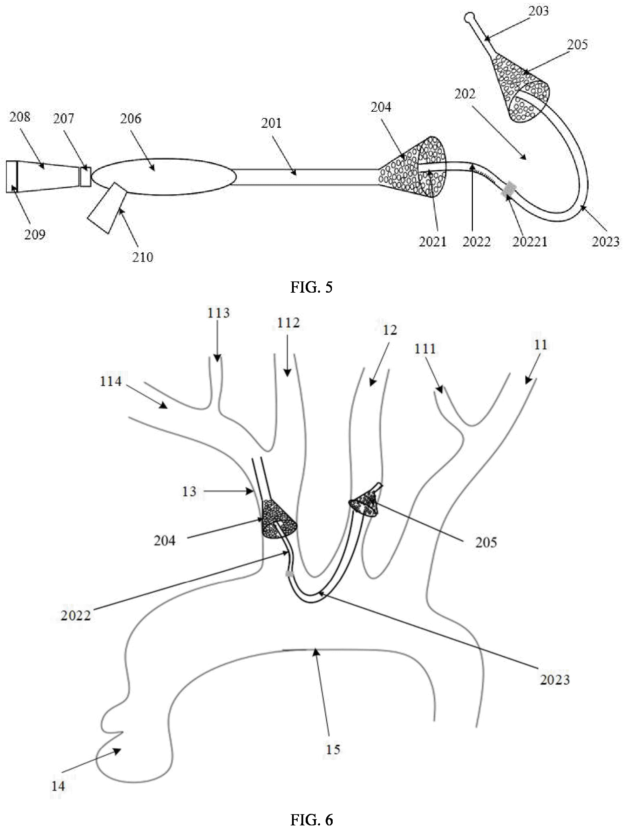

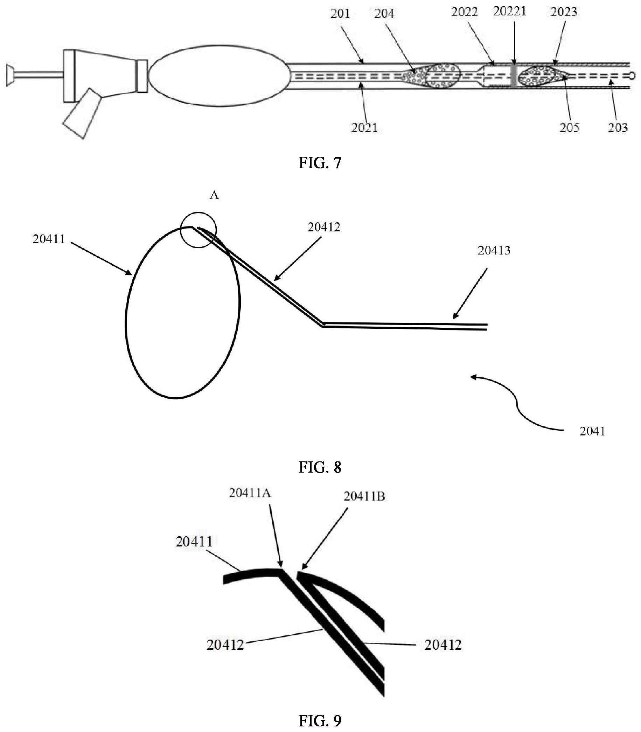

The thrombus filtering device described above has a retrieval state and a use state. In the retrieval state (referring to FIG. 7), the distal filter 205 is retracted into the articulating sheath, and the outer sheath tube 201 is sleeved outside the proximal filter 204, so as to facilitate entering and withdrawing from human blood vessels. In the use state (referring to FIG. 5 ), the distal filter 205 extends out of the articulating sheath, and the outer sheath tube 201 exposes the proximal filter 204, so as to facilitate the proximal filter 204 and the distal filter 205 filtering in corresponding human blood vessels.

The process of implanting the thrombus filtering device described above into a patient's body is introduced below. An initial state of the thrombus filtering device is the retrieval state. The thrombus filtering device is inserted in a radial artery puncture to extend into the right subclavian artery 114 and the brachiocephalic artery 13. Then, by withdrawing the outer sheath tube 201, the proximal filter 204 is exposed from the distal end of the outer sheath tube 201 so as to be positioned in the brachiocephalic artery 13. Further, by moving the inner sheath tube 203 distally relative to the intermediate sheath tube 202 and extending the same into the aortic arch 15, the distal filter 205 is exposed from the distal end of the articulating sheath. Then, by pulling the bend-adjusting pull wire 20231 towards a proximal direction to cause the articulating sheath to bend, the distal filter 205 extending out of the articulating sheath enters from the aortic arch 15 into the left common carotid artery 12 as the articulating sheath bends, while the proximal filter 204 can remain in the brachiocephalic artery 13.

Since the inner sheath tube 203 can move relative to the intermediate sheath tube 202, the relative distance between the distal filter 205 arranged on the inner sheath tube 203 and the proximal filter 204 arranged on the intermediate sheath tube 202 can be adjusted, and thereby the relative distance between the proximal filter 204 and the distal filter 205 can be adjusted. In this way, the thrombus filtering device described above enables the adjustment of the relative distance between the proximal filter 204 and the distal filter 205 according to actual conditions of blood vessels of different patients, so that both the proximal filter 204 and the distal filter 205 can be quickly and accurately positioned at suitable vascular sites, thereby allowing the thrombus filtering device described above to flexibly adapt to arterial vessels of different patients. Moreover, since the inner sheath tube 203 can move relative to the intermediate sheath tube 202, the proximal filter 204 and the distal filter 205 can move relatively independently, thereby facilitating independently adjusting positions of the proximal filter 204 and the distal filter 205, respectively.

When the thrombus filtering device described above is in the use state, the spring tube section 20321 extends out of the distal end of the articulating sheath. When it is withdrawn from the human body after use, it needs to be adjusted to the retrieval state, so that the distal filter 205 needs to be retrieved into the articulating sheath, and therefore the spring tube section 20321 also needs to be retrieved into the articulating sheath. During a process in which the spring tube section 20321 is retrieved into the articulating sheath, it can adapt to bent portions of the articulating sheath through its elastic bending deformation, thereby facilitating retrieval of the inner sheath tube 203 carrying the distal filter 205 into the articulating sheath.

As described above, when the outer sheath tube 201 is pushed distally, the proximal filter 204 can be retrieved inside it; when the outer sheath tube 201 is withdrawn proximally, the proximal filter 204 will be released for embolus filtering. Therefore, the most distal position of the proximal filter 204 does not exceed the farthest distance that the outer sheath tube 201 can be pushed, and likewise, the most proximal position of the proximal end of the proximal filter 204 is not proximal to the closest distance that the outer sheath tube 201 can be withdrawn. Optionally, the distance from the distal end of the proximal filter 204 to the proximal end of the push tube 2021 is greater than or equal to 800 mm, and less than or equal to 1100 mm.

Similarly, when the inner sheath tube 203 is pushed distally, the distal filter 205 can be released for protection from thrombosis; when the inner sheath tube 203 is withdrawn proximally, the distal filter 205 will be retrieved into the articulating sheath. Therefore, the entire length of the distal filter 205 should not be greater than the entire length of the articulating sheath.

Optionally, the proximal delivery tube 2033 is a braided tube with high strength, high toughness, and easy delivery, so as to facilitate delivery of the inner sheath tube 203. Specifically, the proximal delivery tube 2033 can include an inner layer, an outer layer, and a middle layer, and the inner layer and outer layer thereof can be made of polymer materials, such as any one or more of PU, PET, TPU, PTFE, PI, Pebax, etc. The middle layer can be formed by weaving a metal material into a net shape, basket shape, or spring shape through a certain process. The material of the middle layer can be at least one of materials such as stainless steel, nickel-titanium, nickel, etc. The proximal delivery tube 2033 has an inner diameter greater than or equal to 0.2 mm, and less than or equal to 0.5 mm, and an outer diameter greater than or equal to 0.4 mm, and less than or equal to 0.6 mm.

Referring to FIG. 2 to FIG. 7 in combination, in an embodiment, the articulating sheath includes a first tube 2022 and a second tube 2023, a proximal end of the first tube 2022 is connected to the distal end of the push tube 2021, and a distal end of the first tube 2022 is connected to a proximal end of the second tube 2023. The bend-adjusting pull wire 20231 can drive the first tube 2022 and the second tube 2023 to bend sequentially, wherein the first tube 2022 bends toward a first direction, the second tube 2023 bends toward a second direction, and the second direction is opposite to the first direction.

Specifically, the distal end of the bend-adjusting pull wire 20231 can be connected to the second tube 2023, and the bend-adjusting pull wire 20231 extends through the second tube 2023, the first tube 2022, and the push tube 2021. When the bend-adjusting pull wire 20231 is pulled from the proximal end of the bend-adjusting pull wire 20231, the second tube 2023 bends first. Continuing to pull the bend-adjusting pull wire 20231 further, the first tube 2022 will also be bent accordingly, achieving a double-bending effect. The bending directions of the first tube 2022 and the second tube 2023 are opposite. By means of double bending, the articulating sheath can adapt to the spatial shape of the complex physiological structure at the aortic arch 15, thereby quickly and accurately positioning the proximal filter 204 and the distal filter 205 in the brachiocephalic artery 13 and the left common carotid artery 12, respectively.

The first tube 2022 can adopt a keel structure. The second tube 2023 can be made of a polymer material. As shown in FIG. 2 , the first tube 2022 and the second tube 2023 can be connected via a connecting piece 20221.

Referring to FIG. 8 to FIG. 13 in combination, in an embodiment, the proximal filter 204 includes a first elastic metal framework 2041 and a first polymer film 2042. The first polymer film 2042 includes a first porous filter membrane 20421. The first porous filter membrane 20421 covers the first elastic metal framework 2041, and the first elastic metal framework 2041 is configured to support a shape of the first porous filter membrane 20421. The first porous filter membrane 20421 has a collapsed state and an expanded state. In the expanded state, the first porous filter membrane 20421 tapers from a distal end to a proximal end.

The first elastic metal framework 2041 is an elastic framework, so that the proximal filter 204 can be retrieved in the collapsed state inside the outer sheath tube 201 through elastic compression. When released outside the outer sheath tube 201, the proximal filter 204 can return to the expanded state through elasticity of the first elastic metal framework 2041, thereby expanding the first porous filter membrane 20421. The material of the first elastic metal framework 2041 is, for example, at least one of metal materials such as nickel-titanium alloy, chromium-nickel-iron alloy, etc.

When expanded by the first elastic metal framework 2041, i.e., in the expanded state, the first porous filter membrane 20421 tapers from the distal end to the proximal end, so that the distal end of the proximal filter 204 forms an opening, overall presenting a shape such as an umbrella shape or a triangular cone shape, etc. The first porous filter membrane 20421 has holes of a certain shape and a certain area for blood flow and filtering embolic materials.

The proximal filter 204 is fixed at its proximal end to the push tube 2021, and specifically, can be fixed by means of bonding, welding, thermal fusing, etc.

Preferably, the first porous filter membrane 20421 covers an outer side of the first elastic metal framework 2041. In some cases, the first porous filter membrane 20421 can also cover an inner side of the first elastic metal framework 2041.

Different from existing pure metal braided stent-type filters, the proximal filter 204 in the present embodiments adopts elastic metal as a framework, and a polymer porous filter membrane is disposed on the elastic metal framework. Compared with pure metal braided stent-type filters, the pore size of the polymer porous filter membrane is more flexible and controllable, and is lower in processing difficulty. Meanwhile, when the same strength performance requirements are achieved, the thickness of the polymer porous filter membrane is much lower than that of the pure metal braided stent (up to 1/10), thus it has a smaller space-occupying effect during device delivery and retrieval, and is easier to retrieve and deliver.

Moreover, because the polymer porous filter membrane has a small thickness and high strength, it is easier to filter substances such as large-sized thrombi, plaques, etc. in actual use. When filtering out embolic materials of a relatively large size, the structure of the proximal filter 204 of the present embodiments is easier to be retrieved into the outer sheath tube 201 compared with pure metal braided stent-type filters under the same filtering conditions, avoiding the situation of partial retrieval or inability to retrieve, while greatly reducing the pressure on the outer sheath tube 201, the inner sheath tube 203, and the brachiocephalic artery 13 during the retrieval process, thereby greatly improving the safety of the product.

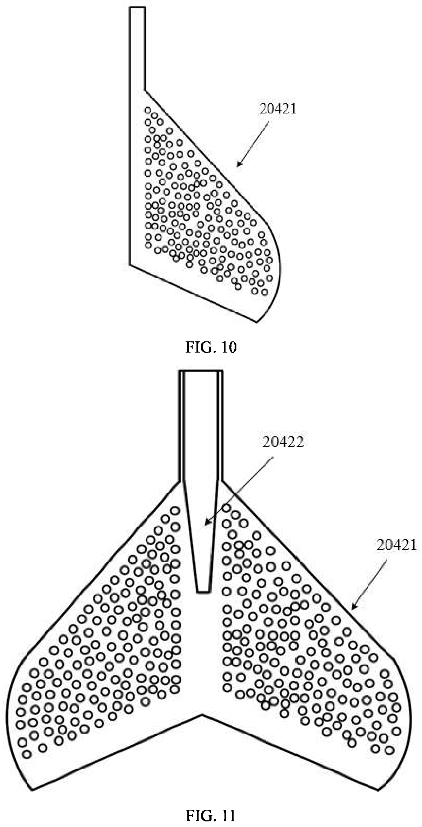

The first porous filter membrane 20421 is made of a polymer material, which can be any one or more of PU, PET, TPU, PTFE, etc. Filter holes with a certain pore size, a certain hole spacing, and a certain area are distributed on the first porous filter membrane 20421. The pore size can be 80 to 160 µm. The hole spacing is generally comparable to the pore size, being 80 to 160 µm. The area of the filter holes cannot be too large, otherwise it will affect the overall strength and filtering performance of the first porous filter membrane 20421, nor can it be too small, otherwise it will affect the passage of the overall blood flow, causing excessively high local blood pressure and an excessively large pressure difference. The area of the filter holes can be two-thirds of the overall membrane area, as shown in FIG. 11.

In an embodiment, a distal outer periphery of the first elastic metal framework 2041 is sleeved by a first radiopaque ring (not shown). The first radiopaque ring can be made of platinum, platinum-tungsten, or platinum-iridium alloy.

Referring to FIG. 8 to FIG. 13 in combination, in an embodiment, the first elastic metal framework 2041 includes a first support ring 20411, a first support wire 20412, and a first fixing wire 20413. The distal end of the first porous filter membrane 20421 is connected to and fitted in shape with the first support ring 20411. The first fixing wire 20413 is attached and fixed to the push tube 2021, a proximal end of the first support wire 20412 is connected to the first fixing wire 20413, and a distal end of the first support wire 20412 is connected to the first support ring 20411.

Optionally, the first support ring 20411, the first support wire 20412, and the first fixing wire 20413 have a wire diameter greater than or equal to 0.10 mm, and less than or equal to 0.35 mm.

Optionally, a size of the first elastic metal framework 2041 along a length direction of the push tube 2021 is greater than or equal to 40 mm, and less than or equal to 70 mm.

The first support ring 20411, in a natural state, can be elliptical. Optionally, a long axis of the first support ring 20411 is greater than or equal to 6 mm, and less than or equal to 12 mm; a short axis is greater than or equal to 5 mm, and less than or equal to 10 mm. The first support ring 20411 in the natural state can also be circular.

The first support ring 20411 can be a closed structure, or an unclosed open ring. As shown in FIG. 8 and FIG. 9 in combination, when the first support ring 20411 is an unclosed open ring, two open ends of the first support ring 20411 are connected to two first support wires 20412, respectively, thereby forming two connection points, namely a connection point 20411A and a connection point 20411B.

In the embodiment shown in FIG. 8 and FIG. 9 , the number of the first support wires 20412 is two. In other embodiments, the number of the first support wires 20412 is not limited to two, and can also be one, three, four, etc.

The first support wire 20412 and an axial end face of the first support ring 20411 are arranged at an angle, and the angle therebetween can be 60° to 120°.

A first reference plane and a first projection plane are defined. The first reference plane is a plane determined by the central axis of the first support ring 20411 and the central axis of the first support wire 20412. The first projection plane is a plane perpendicular to the first reference plane and extending through the central axis of the first support ring 20411. Optionally, a length of a projection of the first support wire 20412 on the first projection plane is greater than or equal to 15 mm, and less than or equal to 25 mm.

As shown in FIG. 8 , the first fixing wire 20413 and the first support wire 20412 are connected in one-to-one correspondence. The first fixing wire 20413 can extend along a linear direction and be fixed tightly against the push tube 2021. The first fixing wire 20413 can also be wound around the push tube 2021. Optionally, a length of a projection of the first fixing wire 20413 on the first projection plane is greater than or equal to 25 mm, and less than or equal to 35 mm.

Specifically, the first radiopaque ring can be sleeved on and connected to the first support ring 20411, and two ends of the first radiopaque ring are aligned with the first support ring 20411 to maintain its stability.

Referring to FIG. 11 , in an embodiment, the first polymer film 2042 further includes a support membrane 20422. The support membrane 20422 is arranged around and attached to the proximal end of the first porous filter membrane 20421.

Specifically, the proximal end of the first porous filter membrane 20421 is tube-shaped for sleeving outside the push tube 2021. The support membrane 20422 surrounds and is attached to the outer periphery of the tube-shaped structure at its proximal end, so as to protect the proximal end of the first porous filter membrane 20421, thereby avoiding wear of the proximal filter 204 during movement as much as possible.

The support membrane 20422 is made of a polymer material, and can be any one or more of PU, PET, TPU, PTFE, etc.

Optionally, the support membrane 20422 has a length greater than or equal to 15 mm, and less than or equal to 35 mm, and has a width greater than or equal to 1 mm, and less than or equal to 5 mm.

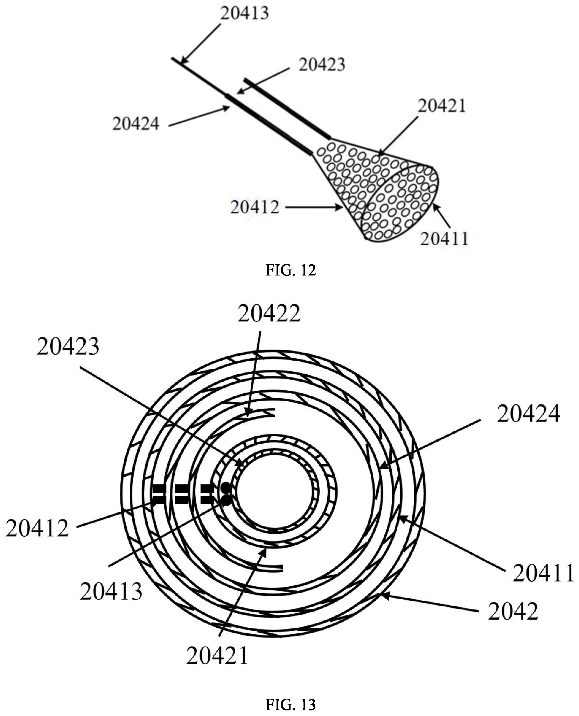

Referring to FIG. 12 and FIG. 13 in combination, in an embodiment, the first polymer film 2042 further includes an inner sleeve section 20423, the inner sleeve section 20423 is sleeved on and attached to the push tube 2021, and the proximal end of the first porous filter membrane 20421 is sleeved on and attached to the inner sleeve section 20423. Thus, the inner sleeve section 20423 is located at the innermost layer of the proximal filter 204. The inner sleeve section 20423 is configured to facilitate connection of the proximal end of the first porous filter membrane 20421 with the push tube 2021. Optionally, the inner sleeve section 20423 has an inner diameter greater than or equal to 1 mm, and less than or equal to 1.2 mm, an outer diameter greater than or equal to 1.1 mm, and less than or equal to 1.4 mm, and a length greater than or equal to 8 mm, and less than or equal to 12 mm.

Referring to FIG. 12 and FIG. 13 in combination, in an embodiment, the first polymer film 2042 further includes an outer sleeve section 20424, and the outer sleeve section 20424 is sleeved on and attached to the proximal end of the first porous filter membrane 20421. Thus, the outer sleeve section 20424 is located at the outermost side of the entire proximal filter 204, for protecting and fastening the proximal end of the proximal filter 204. The outer sleeve section 20424 has an inner diameter greater than or equal to 1.4 mm, and less than or equal to 1.6 mm, an outer diameter greater than or equal to 1.5 mm, and less than or equal to 1.8 mm, and a length greater than or equal to 8 mm, and less than or equal to 20 mm.

In an embodiment, when the thrombus filtering device described above is in the use state, the spring tube section 20321 extends out of the second tube 2023. During a process in which the spring tube section 20321 is retrieved into the second tube 2023, it can adapt to bent portions of the second tube 2023 through its elastic bending deformation, thereby facilitating retrieval of the inner sheath tube 203 carrying the distal filter 205 into the articulating sheath. The spring tube section 20321 has a length greater than or equal to 5 mm, and less than or equal to 10 mm.

Referring to FIG. 14 to FIG. 16 in combination, in an embodiment, the distal fixing tube 2032 includes a cutting tube section 20323 The cutting tube section 20323 is configured to fix the distal filter 205, thereby facilitating fixing the distal filter 205 with the distal fixing tube 2032. A plurality of cutting slots 20324 sequentially arranged along the axial direction are provided on the cutting tube section 20323.

When the thrombus filtering device described above is in the use state, the cutting tube section 20323 extends out of the distal end of the articulating sheath. When it needs to be withdrawn from the human body after use, it needs to be adjusted to the retrieval state, so that the distal filter 205 needs to be retrieved into the articulating sheath, and therefore the cutting tube section 20323 also needs to be retrieved into the articulating sheath.

By providing the plurality of cutting slots 20324 on the cutting tube section 20323, the strength of the cutting tube section 20323 can be reduced. Therefore, during the process in which the cutting tube section 20323 is retrieved into the articulating sheath, the cutting tube section 20323 is made easy to bend so as to adapt to the bent portions of the articulating sheath, thereby facilitating retrieval of the inner sheath tube 203 carrying the distal filter 205 into the articulating sheath. Optionally, the cutting tube section 20323 has a length greater than or equal to 3 mm, and less than or equal to 5 mm. The proximal delivery tube 2033 has a length greater than or equal to 1200 mm, and less than or equal to 1600 mm.

Referring to FIG. 14 to FIG. 16 in combination, in an embodiment, a distal end of the cutting tube section 20323 is connected to a proximal end of the spring tube section 20321.

Specifically, the distal fixing tube 2032 includes a connecting portion 20322, and the distal end of the cutting tube section 20323 and the proximal end of the spring tube section 20321 can be connected via the connecting portion 20322.

Referring to FIG. 16 , FIG. 18 to FIG. 21, in an embodiment, the distal filter 205 includes a second elastic metal framework 2051 and a second polymer film 2052. The second polymer film 2052 includes a second porous filter membrane 20521. The second porous filter membrane 20521 is connected to the second elastic metal framework 2051. The second porous filter membrane 20521 has a collapsed state and an expanded state, the second elastic metal framework 2051 is configured to support a shape of the second porous filter membrane 20521, and in the expanded state, the second porous filter membrane 20521 tapers from a proximal end to a distal end direction.

The second elastic metal framework 2051 is an elastic framework, so that the distal filter 205 can be retrieved in the collapsed state inside the intermediate sheath tube 202 through elastic compression. When released outside the intermediate sheath tube 202, the distal filter 205 can return to the expanded shape through elasticity of the second elastic metal framework 2051, thereby expanding the second porous filter membrane 20521. The material of the second elastic metal framework 2051 is, for example, at least one of metal materials such as nickel-titanium alloy, chromium-nickel-iron alloy, etc.

When expanded by the second elastic metal framework 2051, i.e., in the expanded state, the second porous filter membrane 20521 tapers from the proximal end to the distal end, so that the distal end of the distal filter 205 forms an opening, overall presenting a shape such as an umbrella shape or a triangular cone shape, etc. The second porous filter membrane 20521 has holes of a certain shape and a certain area for blood flow and filtering embolic materials.

The distal filter 205 is fixed at both its distal end and proximal end to the inner sheath tube 203, and specifically, can be fixed by means of bonding, welding, thermal fusing, etc.

Preferably, the second porous filter membrane 20521 covers an outer side of the second elastic metal framework 2051. In some cases, the second porous filter membrane 20521 can also cover an inner side of the second elastic metal framework 2051.

Different from existing pure metal braided stent-type filters, the distal filter 205 in the present embodiments uses elastic metal as a framework, and a polymer porous filter membrane is disposed on the elastic metal framework. Compared with pure metal braided stent-type filters, the pore size of the polymer porous filter membrane is more flexible and controllable, and is lower in processing difficulty. Meanwhile, when the same strength performance requirements are achieved, the thickness of the polymer porous filter membrane is much lower than that of the pure metal braided stent (up to 1/10), thus it has a smaller space-occupying effect during device delivery and retrieval, and is easier to retrieve and deliver.

Moreover, because the polymer porous filter membrane has a small thickness and high strength, it is easier to filter substances such as large-sized thrombi, plaques, etc. in actual use. When filtering out embolic materials of a relatively large size, the structure of the distal filter 205 of the present embodiments is easier to be retrieved into the intermediate sheath tube 202 compared with pure metal braided stent-type filters under the same filtering conditions, avoiding the situation of partial retrieval or inability to retrieve, while greatly reducing the pressure on the intermediate sheath tube 202, the inner sheath tube 203, and the left common carotid artery 12 during the retrieval process, thereby greatly improving the safety of the product.

The second porous filter membrane 20521 is made of a polymer material, which can be any one or more of PU, PET, TPU, PTFE, etc. Filter holes with a certain pore size, a certain hole spacing, and a certain area are distributed on the second porous filter membrane 20521. The pore size can be 80 to 160 µm The hole spacing is generally comparable to the pore size, being 80 to 160 µm. The area of the filter holes cannot be too large, otherwise it will affect the overall strength and filtering performance of the second porous filter membrane 20521, nor can it be too small, otherwise it will affect the passage of the overall blood flow, causing excessively high local blood pressure and an excessively large pressure difference.

In an embodiment, a distal outer periphery of the second elastic metal framework 2041 is sleeved by a second radiopaque ring (not shown). The material of the second radiopaque ring can be at least one of platinum, platinum-tungsten, or platinum-iridium alloy materials.

Referring to FIG. 15 to FIG. 17 in combination, in an embodiment, the inner sheath tube 203 includes a tip tube 2031. The tip tube 2031 is a polymer tube, and the tip tube 2031 is connected to a distal end of the distal fixing tube 2032. A distal end of the second elastic metal framework 2051 is connected to a proximal end of the second porous filter membrane 20521 and supports a shape of the proximal end of the second porous filter membrane 20521, a distal end of the second porous filter membrane 20521 is connected to the tip tube 2031, and the second elastic metal framework 2051 is connected to the distal fixing tube 2032.

In this way, the second elastic metal framework 2051 supports the proximal end of the second porous filter membrane 20521, and the tip tube 2031 supports the distal end of the second porous filter membrane 20521, so that the second porous filter membrane 20521, in the expanded state, presents a shape gradually tapering from the proximal end to the distal end.

The second elastic metal framework 2051 is further connected to the distal fixing tube 2032. Thus, when the distal filter 205 needs to be retrieved into the articulating sheath, during the process in which the inner sheath tube 203 moves proximally, the distal fixing tube 2032 can pull the second elastic metal framework 2051 to move proximally, so that the distal end of the second elastic metal framework 2051 enters the articulating sheath through elastic compression, and then the distal end of the second elastic metal framework 2051 simultaneously compresses and pulls the second porous filter membrane 20521 into the articulating sheath.

The tip tube 2031 can be a polymer tube, such as any one or more of PU, PET, TPU, PTFE, Pebax, etc.. The tip tube 2031 has an inner diameter greater than or equal to 0.2 mm, and less than or equal to 0.5 mm, an outer diameter greater than or equal to 0.4 mm, and less than or equal to 0.5 mm, and a length greater than or equal to 30 mm, and less than or equal to 60 mm.

In combination with FIG. 15 to FIG. 17 , in an embodiment, the proximal end of the tip tube 2031 is embedded into the spring tube section 20321 and connecting portion 20322 of the distal fixing tube 2032, and the distal end of the proximal delivery tube 2033 is embedded into the cutting tube section 20323 of the distal fixing tube 2032. Therefore, compared with the tip tube 2031 and the proximal delivery tube 2033, the distal fixing tube 2032 has the largest inner and outer diameter sizes. Optionally, the distal fixing tube 2032 has an inner diameter greater than or equal to 0.4 mm, and less than or equal to 0.6 mm, an outer diameter greater than or equal to 0.5 mm, and less than or equal to 1 mm, and a length greater than or equal to 10 mm, and less than or equal to 20 mm.

Referring to FIG. 16, in an embodiment, the second polymer film 2052 includes a tip cap 2053. The tip cap 2053 is connected to the distal end of the second porous filter membrane 20521, and the tip cap 2053 wraps the distal end of the tip tube 2031. In this way, when assembling the second polymer film 2052 and the tip tube 2031, the second polymer film 2052 can be covered on the distal end of the tip tube 2031 through the tip cap 2053, and then the proximal end of the second porous filter membrane 20521 is connected to the proximal end of the second elastic metal framework 2051, so that the second porous filter membrane 20521 can be supported by the proximal end of the second elastic metal framework 2051 and the distal end of the tip tube 2031. Thus, the distal end of the distal filter 205 and the distal end of the inner sheath tube 203 are connected through a polymer material, without the need to provide a metal material, so that the distal end of the distal filter 205 and the distal end of the inner sheath tube 203 are made of relatively soft materials, which are easy to bend and are not likely to damage the blood vessel. The tip cap 2053 and the distal end of the tip tube 2031 can be connected by means of bonding, fusing, etc.

Referring to FIG. 16 to FIG. 21 , in an embodiment, the second elastic metal framework 2051 includes a second support ring 20511, a second support wire 20512, and a second fixing wire 20513. The proximal end of the second porous filter membrane 20521 is connected to and fitted in shape with the second support ring 20511. The second fixing wire 20513 is attached and fixed to the distal fixing tube 2032, a proximal end of the second support wire 20512 is connected to the second fixing wire 20513, and a distal end of the second support wire 20512 is connected to the second support ring 20511.

The proximal end of the second porous filter membrane 20521 surrounds the second support ring 20511 circumferentially, and is supported by the second support ring 20511, so that the shape of the proximal end of the second porous filter membrane 20521 can be maintained.

Since the proximal filter 204 and the distal filter 205 are positioned in different vascular sites during use, they also differ in size.

Optionally, the second support ring 20511, the second support wire 20512, and the second fixing wire 20513 have a wire diameter greater than or equal to 0.05 mm, and less than or equal to 0.2 mm.

A size of the second elastic metal framework 2051 along a length direction of the inner sheath tube 203 is greater than or equal to 30 mm, and less than or equal to 60 mm.

The second support ring 20511, in a natural state, can be elliptical. Optionally, a long axis is greater than or equal to 4 mm, and less than or equal to 10 mm, and a short axis is greater than or equal to 3 mm, and less than or equal to 6 mm. In some cases, the second support ring 20511 in the natural state can also be circular.

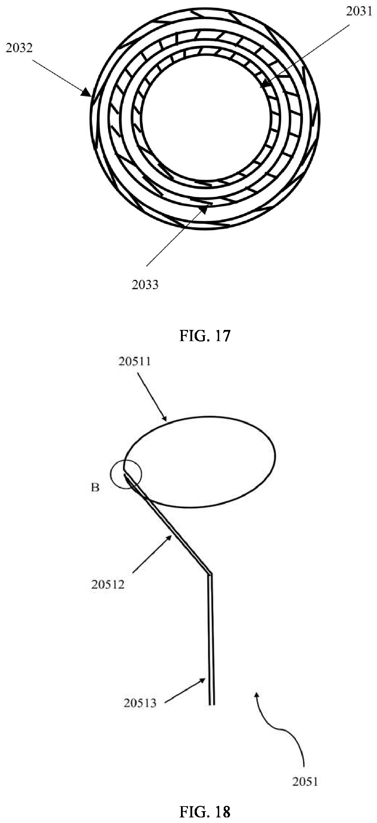

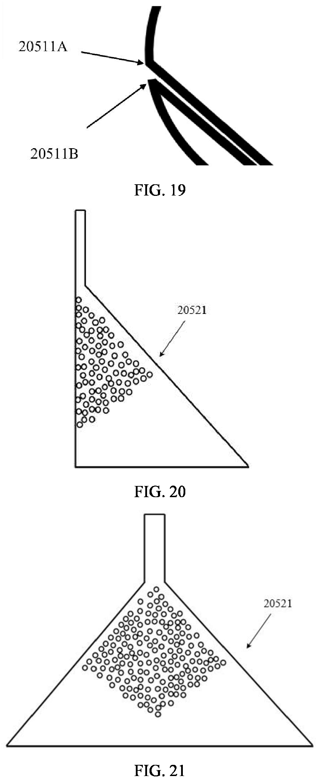

The second support ring 20511 can be a closed structure, or an unclosed open ring. As shown in FIG. 18 and FIG. 19 in combination, when the second support ring 20511 is an unclosed open ring, two open ends of the second support ring 20511 are connected to two second support wires 20512, respectively, thereby forming two connection points, namely a connection point 20511A and a connection point 20511B.

In the embodiment shown in FIG. 18 and FIG. 19 , the number of the second support wires 20512 is two. In other embodiments, the number of the second support wires 20512 is not limited to two, and can also be one, three, four, etc.

The second support wire 20512 and an axial end face of the second support ring 20511 are arranged at an angle, and the angle therebetween can be 60° to 120°.

A second reference plane and a second projection plane are defined. The second reference plane is a plane determined by the central axis of the second support ring 20511 and the central axis of the second support wire 20512. The second projection plane is a plane perpendicular to the second reference plane and extending through the central axis of the second support ring 20511. Optionally, a length of a projection of the second support wire 20512 on the second projection plane is greater than or equal to 10 mm, and less than or equal to 20 mm.

As shown in FIG. 18 and FIG. 19 , the second fixing wire 20513 and the second support wire 20512 are connected in one-to-one correspondence. The second fixing wire 20513 can extend along a linear direction and be fixed tightly against the inner sheath tube 203.

The second fixing wire 20513 can also be wound around the inner sheath tube 203. Optionally, a length of a projection of the second fixing wire 20513 on the second projection plane is greater than or equal to 15 mm, and less than or equal to 25 mm.

Specifically, the second radiopaque ring can be sleeved on and connected to the second support ring 20511, and two ends of the second radiopaque ring are aligned with the second support ring 20511 to maintain its stability.

In an embodiment, the thrombus filtering device further includes an operation assembly. The proximal end of the outer sheath tube 201, the proximal end of the intermediate sheath tube 202, and the proximal end of the inner sheath tube 203 are respectively connected to the operation assembly. The operation assembly is configured to control movement of the outer sheath tube 201 relative to the intermediate sheath tube 202, and to control movement of the inner sheath tube 203 relative to the intermediate sheath tube 202, and also to control bending of the articulating sheath. The proximal end of the bend-adjusting pull wire can be connected to the operation assembly.

In combination with FIG. 2, FIG. 4 and FIG. 5, in an embodiment, the operation assembly includes a first handle 206, a sheath pushing knob 207, a second handle 208, a filter pushing knob 209, and a bend-adjusting control knob 210. The first handle 206 is connected to the outer sheath tube 201, and is configured to control movement of the outer sheath tube 201 relative to the intermediate sheath tube 202. The second handle 208 is configured for an operator to hold. The filter pushing knob 209 is configured to control movement of the inner sheath tube 203 relative to the intermediate sheath tube 202. The bend-adjusting control knob 210 is connected to the proximal end of the bend-adjusting pull wire, and is configured to control bending of the articulating sheath. The various components of the operation assembly can be directly connected or indirectly connected through intermediate members, as long as the movement and control functions of the various components thereof can be met, and no limitation is imposed on this.

The specific structure of the operation assembly can also refer to other operation assemblies in the prior art, as long as relative movement of the three tubes, i.e., the outer sheath tube 201, the intermediate sheath tube 202, and the inner sheath tube 203, and bending of the articulating sheath of the intermediate sheath tube 202 can be achieved.

The embodiments in this specification are all described in a progressive manner. Description of each of the embodiments focuses on differences from other embodiments, and reference may be made to each other for the same or similar parts among respective embodiments.

The above description of the disclosed embodiments enables those skilled in the art to implement or use the present application. Various modifications to these embodiments are obvious to those skilled in the art, and the general principles defined herein may be implemented in other embodiments without departing from the spirit or scope of the present application. Therefore, the present application is not to be limited to the embodiments shown herein, but the broadest scope consistent with the principle and novel features disclosed herein is to be accorded.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A thrombus filtering device, comprising: an outer sheath tube, an intermediate sheath tube, a proximal filter, an inner sheath tube, and a distal filter;
wherein the intermediate sheath tube comprises a push tube and an articulating sheath, a distal end of the push tube is connected to a proximal end of the articulating sheath, the proximal filter is connected to the push tube, and the outer sheath tube is movably sleeved outside the intermediate sheath tube; the distal filter is connected to a distal end of the inner sheath tube, and the inner sheath tube is movably inserted in the intermediate sheath tube;
the inner sheath tube comprises a proximal delivery tube and a distal fixing tube which are connected to each other, the distal fixing tube is a metal tube, and the distal fixing tube comprises a spring tube section.

2. The thrombus filtering device according to claim 1, wherein the distal fixing tube comprises a cutting tube section, the cutting tube section is provided with a plurality of cutting slots sequentially arranged along an axial direction.

3. The thrombus filtering device according to claim 1, wherein the proximal filter comprises a first elastic metal framework and a first polymer film, the first polymer film comprises a first porous filter membrane that covers the first elastic metal framework, the first porous filter membrane has a collapsed state and an expanded state, and in the expanded state, the first porous filter membrane tapers along a direction from a distal end to a proximal end; and/or
the distal filter comprises a second elastic metal framework and a second polymer film, the second polymer film comprises a second porous filter membrane that is connected to the second elastic metal framework, the second porous filter membrane has a collapsed state and an expanded state, and in the expanded state, the second porous filter membrane tapers along a direction from a proximal end to a distal end.

4. The thrombus filtering device according to claim 3, wherein the first elastic metal framework comprises a first support ring, a first support wire, and a first fixing wire, wherein a distal end of the first porous filter membrane is connected to and fitted in shape with the first support ring, the first fixing wire is attached and fixed to the push tube, a proximal end of the first support wire is connected to the first fixing wire, and a distal end of the first support wire is connected to the first support ring.

5. The thrombus filtering device according to claim 3, wherein the first polymer film further comprises a support membrane, the support membrane is disposed around and attached to a proximal end of the first porous filter membrane.

6. The thrombus filtering device according to claim 3, wherein the first polymer film further comprises an inner sleeve section, the inner sleeve section is sleeved on and attached to the push tube, and the proximal end of the first porous filter membrane is sleeved on and attached to the inner sleeve section.

7. The thrombus filtering device according to claim 3, wherein the first polymer film further comprises an outer sleeve section, the outer sleeve section is sleeved on and attached to the proximal end of the first porous filter membrane.

8. The thrombus filtering device according to claim 3, wherein the inner sheath tube comprises a tip tube, the tip tube is a polymer tube, and a proximal end of the tip tube is connected to a distal end of the distal fixing tube;
a distal end of the second elastic metal framework is connected to a proximal end of the second porous filter membrane and supports a shape of the proximal end of the second porous filter membrane, a distal end of the second porous filter membrane is connected to the tip tube, and the second elastic metal framework is connected to the distal fixing tube.

9. The thrombus filtering device according to claim 8, wherein the second polymer film comprises a tip cap, the tip cap is connected to the distal end of the second porous filter membrane, and the tip cap wraps a distal end of the tip tube.

10. The thrombus filtering device according to claim 3, wherein the second elastic metal framework comprises a second support ring, a second support wire, and a second fixing wire, the proximal end of the second porous filter membrane is connected to and fitted in shape with the second support ring; the second fixing wire is attached and fixed to the distal fixing tube, a proximal end of the second support wire is connected to the second fixing wire, and a distal end of the second support wire is connected to the second support ring.

11. The thrombus filtering device according to claim 1, wherein the thrombus filtering device further comprises a bend-adjusting pull wire, a distal end of the bend-adjusting pull wire is connected to the articulating sheath, and the bend-adjusting pull wire extends through the articulating sheath and the push tube.

12. The thrombus filtering device according to claim 1, wherein a most distal position of the proximal filter does not exceed a farthest distance to which the outer sheath tube is enabled to be pushed, and a most proximal position of the proximal end of the proximal filter is no closer than a closest distance to which the outer sheath tube is enabled to be retrieved.

13. The thrombus filtering device according to claim 11, wherein the articulating sheath comprises a first tube and a second tube, a proximal end of the first tube is connected to the distal end of the push tube, a distal end of the first tube is connected to a proximal end of the second tube; the bend-adjusting pull wire is capable of driving the first tube and the second tube to bend sequentially, the first tube bends toward a first direction, the second tube bends toward a second direction, and the second direction is opposite to the first direction.

14. The thrombus filtering device according to claim 13, wherein the distal end of the bend-adjusting pull wire is connected to the second tube, and the bend-adjusting pull wire extends through the second tube, the first tube, and the push tube.

15. The thrombus filtering device according to claim 8, wherein the distal fixing tube further comprises a connecting portion and a cutting tube section, a distal end of the cutting tube section is connected to a proximal end of the spring tube section via the connecting portion,
the proximal end of the tip tube is embedded into the spring tube section and the connecting portion of the distal fixing tube, and the distal end of the proximal delivery tube is embedded into the cutting tube section of the distal fixing tube.
